# EUROPEAN PATENT SPECIFICATION

(11) **EP 0 585 554 B1**
(45) Date of publication and mention of the grant of the patent: **20.11.1996**
(21) Application number: 93110445.9
(22) Date of filing: 30.06.1993
(51) Int. Cl.: C12N 15/82, C07K 14/42, C12N 1/21, C12P 21/02

(54) **Process for preparing a transgenic plant expressing phytolacca antiviral protein**
Verfahren zur Herstellung einer transgenen Pflanze, die das antivirale Protein aus Phytolacca exprimiert
Procédé de préparer une plant transgène qui exprime la protéine antivirale de phytolacca

(30) Priority: 16.08.1992 KR 9214895
(43) Date of publication of application: 09.03.1994
(73) Proprietor: JINRO LIMITED, Seoul (KR)
(72) Inventor: Kim, Man-Keun, Youngdeungpo-Ku, Seoul (KR); Lee, Kwan-Ho, Seocho-Ku, Seoul (KR); Na, Byeong-Kook, Nam-Ku, Incheon (KR); Jeong, Han Seung, Dongjak-Ku, Seoul (KR); Choi, Kyu-Whan, Kangseo-Ku, Seoul (KR); Moon, Young-Ho, Kuri, Kyunggi-Do (KR); Jeon, Hong-Seob, Nowon-Ku, Seoul (KR)
(74) Representative: TER MEER - MÜLLER - STEINMEISTER & PARTNER

(56) References cited:
- EP-A- 0 437 320
- US-A- 4 921 841
- PLANT MOLECULAR BIOLOGY, vol. 17, 1991, pages 609-614; Q. LIN ET AL.
- PROCEEDINGS OF THE NATIONAL ACADEMY OF SCIENCES OF THE USA, vol. 83, 1986, pages 5053-5056; M.P. READY ET AL.: 'Extracellular localization of pokeweed antiviral protein'

## Description

### Field of the Invention

The present invention relates to an expression vector for antiviral protein, more specifically, a recombinant expression vector for phytolacca antiviral protein isolated from Phytolacca americana L. and a process for preparing a transgenic plant transformed thereof.

### Background of the Invention

Since plants cannot escape from the applied pathogens because of their immobility, they must be able to defend themselves by direct or indirect response to the pathogenic challenge; and, most plants appear to undertake some general defense mechanism to protect themselves against applied pathogens, e.g., fungi, bacteria and virus.

In this connection, crude extract isolated from Phytolacca americana L has been reported to inhibit in vivo polypeptide synthesis [see; Owens, R.A. et al., Virology, 56:390-393(1973)]; and, said report has accelerated studies on the phytolacca antiviral protein("PAP") isolated from Phytolacca americana L. Under the circumstance, PAPs such as PAP-I and PAP-II produced in spring and summer, respectively, and PAP-S produced from seed, have been discovered and isolated since early 1970's [see: Irvin; J.D. et al., Arch. Biochem. Biophys., 169:522-528(1975); Irvin, J.D. et al., Arch. Biochem. Biophys., 200:418-425(1980); Barbieri, I. et al., Biochem. J., 203:55-59(1982)].

On the other hand, as a result of extensive studies on the PAP in molecular level, it was determined that PAPs inactivate 60S ribosomal subunit of eucaryotic polypeptide synthesis machinery, which is a general phenomenon as other ribosome-inactivating proteins(RIPs) inactivate said subunit[see: Houston, L.L. et al., J. Biol. Chem., 258:9601-9604(1983)]. Further, it has been reported that: PAP is synthesized and secreted from the cytosol and involved in the control of pathogenic virus; however, the detailed mechanism of virus inactivation has not been proved[see: Ready, M.P. et al., Proc. Natl. Acad. Sci., 83:5053-5056(1986)].

Structure and base sequence of PAP genome, a multigene family, have been characterized and determined [see: Lin, Q. et al., Plant Mol. Biol., 17:609-614(1991)]; and, therefore, efforts have continued to exist in the art, for the development of expression vector for PAP and transgenic plant transformed thereof.

### Summary of the Invention

In accordance with the present invention, it has been discovered that: PAP is manufactured in recombinant expression vector.

A primary object of the present invention is, therefore, to provide a novel recombinant vector containing PAP gene isolated from cDNA library of the Phytolacca americana L.

Another objects of the present invention are to provide a process for preparing PAP, an antiviral protein, from transgenic plant cell transformed with said recombinant vector, and to provide a method of elevating the plant's resistance for infective virus.

### Brief Description of Drawings

The above and the other objects and features of the present invention will become apparent from following descriptions given in conjunction with the accompanying drawings, in which:
- Figure 1: is full nucleotide sequence of PAP genome isolated from cDNA library;
- Figure 2: is a stepwise construction scheme of expression vector pJRM100;
- Figure 3: is a photograph showing agarose gel electrophoresis pattern of expression vector pJRM100 digested with restriction enzyme;
- Figure 4: is a photograph showing transgenic tobacco plant cell transformed with pJRM100;
- Figure 5: is a photograph showing the immunodiffusion assay result of purified PAP;
- Figure 6: is a photograph showing the Southern blot analysis of DNA isolated from transgenic tobacco plant;
- Figure 7: is a photograph showing the Southern blot analysis of RNA isolated from transgenic tobacco plant; and,
- Figure 8: is a photograph showing the results of local lesion assay of transgenic tobacco plant.

### Detailed Description of the Invention

The present inventors developed a recombinant expression vector pJRM100 containing PAP gene isolated from cDNA library of Phytolacca americana L. and a transgenic plant transformed thereof by employing a mediator, Agrobacterium tumefaciens.

To isolate PAP gene, the inventors purified total cellular mRNA from leaves of Phytolacca americana L. obtained in Korea and constructed cDNA library thereof. PAP gene is selected by immunoscreening method employing anti-PAP antibody; and, deletion mutant is prepared from the isolated PAP gene using Erase-a-Base system(Promega, U.S.A.). DNA sequence of PAP genome is determined in accordance with Sanger's dideoxy chain termination method[see: Sanger, F., Science, 214:1205-1210(1981)].

For the expression of isolated PAP gene, 5' site of PAP gene is deleted to the site of translational initiation(i.e., ATG) and ligated to BamHI linker; and, 3' site is cleaved with HindIII, treated with Klenow's fragment and ligated to BamHI linker. pJRM100 for the expression of PAP is constructed by ligating BamHI fragments to a binary vector pBI121(Clonetech, Lot #6019-2). Said novel recombinant vector, pJRM100, is deposited with the Korean Collection for Type Culture(KCTC), an international depository authority(IDA) on August 7, 1992 as deposition No. KCTC 0052BP.

Agrobacterium tumefaciens LBA 4404, a mediator for plant cell transformation is transformed with the pJRM100 and transformation of plant cell with said organism is followed. Shoot is induced on MS selective medium containing 200mg/ml kanamycin and 500mg/ml carbenicillin; and root is induced from said shoot. Plant thus obtained is transferred to pot for continuous growth. Immunodiffusion assay is employed to investigate the PAP gene expression; and, Southern and Northern blot methods are employed to investigate proper insertion and transcription of PAP gene, respectively. Resistance of transgenic plant for viral infectivity is tested by local leision assay.

The present invention is further illustrated in the following examples, which should not be taken to limit the scope of the invention.

### Example 1: Isolation of PAP genome.

PAP was isolated in pure culture in accordance with Irvin's method[see: J.D. Irvin, et al., Arch. Biochem. Biophys., 169: 522-528(1975)]. 480µg of PAP was dissolved in PBS solution and combined with Freund's complete adjuvant with a ratio of 1:1 (v/v), and mixture thereof was administrated to a rabbit (∼3kg of body weight) intramuscularly. After 3 weeks, antibody formation was detected in a small amount of blood collected from the rabbit, and 750µg of PAP was combined with Freund's complete adjuvant for boosting with a ratio of 1:1(v/v). Plasma fraction was fractionated from blood by centrifugation; and Protein-A agarose column chromatography was employed to isolate anti-plasma. Immunodiffusion assay and electrophoresis technique were employed to determine antibody formation and homogeniety of isolated PAP, respectively. Purified PAP antibody was stored at -70°C and employed to isolate PAP gene from cDNA library.

To isolate mRNA from the leaves of Phytolacca americana, the leaf tissue was homogenated using liquid nitrogen and, to the homogenate thus prepared was added buffer solution for total RNA isolation; and centrifugation was followed. Total cellular RNA was isolated from supernatant by LiCl sedimentation. mRNA was isolated from total RNA using oligo(dT) cellulose column chromatography, and isolated mRNA was employed for DNA synthesis.

1st strand DNA was synthesized from template mRNA employing M-MuLV reverse transcriptase; and, 2nd strand DNA synthesis by E.coli DNA polymerase was followed. Synthesized cDNA linked to EcoRI adaptor, was subjected to the fractionation by Sephacryl S-400 spun column in accordance with molecular size of cDNA. Fractionated cDNA was ligated to Uni-Zap XR vector(Stratagene Co., U.K.), and in vitro packaging using packaging extract was followed. Immunodiffusion assay employing anti-PAP antibody was carried out to isolate PAP gene from cDNA library thus prepared. Competent E. coli XLl-Blue was infected with phage on petri dish, to form plaque of 2 x 10⁴ pfu. Said bacteria was incubated at 37°C for 15min, and incubation at 42°C for 3.5 hours was followed after plating with 3ml of top agarose. Then, plate was covered with Hybond-N+(Amersham) and further incubated for 5 hours. After serial incubation, Hybond-N+ was blocked with bovine serum albumin and 5mg/ml of anti-PAP antibody was combined. After the removal of free anti-PAP antibody which was not bound with PAP, peroxidase conjugated 2nd antibody was reacted with anti-PAP antibody; and, antigen-antibody complexes thus formed were detected by chloronaphthol treatment.

2nd immunoscreening procedure was followed with 15clones obtained from the 1st immunoscreening method, in a similar fashion as above excluding that plaque number was 5 x 10³ pfu. 3rd immunoscreening procedure was performed with the clones obtained from the 2nd immunoscreening, and 8 plaques isolated were subjected to further experiment.

To transfer phagemids of 8 recombinant Uni-Zap XR phages obtained from the 3rd immunoscreening method, in vivo excision technique employing R408 helper phage was carried out. Plasmids were isolated from the 4 colonies thus selected by alkali denaturation method [see: Maniatis et al., Molecular Cloning: A Laboratory Manual, pp 368-369, Cold Spring Harbor Laboratory (1982)], and colonies harboring PAP gene were screened by restriction enzyme excision method.

Of the clones thus screened, plasmids from 2 clones were isolated, and PAP cDNA insert was sequenced by dideoxy chain termination method. To determine full base sequence of PAP gene, DNA was purified from microorganisms harboring PAP gene. DNA thus purified was digested with SacII and BamHI and deletion was made by intermittent ExoIII excision reaction using Erase-a-Base system(Promega, USA). Deleted DNA was ligated by T₄ DNA ligase and transformation with competent XL1-BLUE cell prepared by CaCl₂ solution was followed. Deletion mutant fractionated by molecular size was employed to determine DNA sequence.

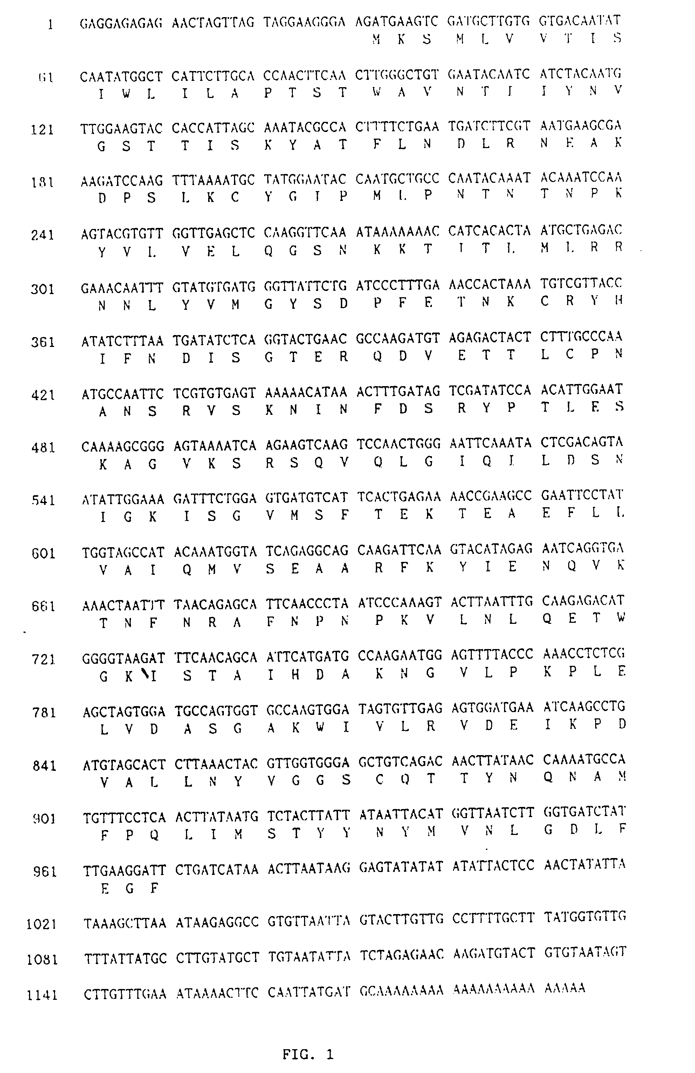

After single strand preparation by alkali denaturation method, full DNA sequence of PAP genome was determined by SEQUENASE VERSION 2.0(United States Biochemical, USA) employing primer such as T₇ promotor primer or universal reverse primer. As disclosed in fig. 1, PAP genome comprises 1195bp of one open reading frame; and cDNA insert of PAP codes 313 of amino acid residues, 22 residues of which functions as signal peptide. Polyadenylation signal(AATAAA) which is ubiquitous in mRNA of most plants and animals, appears to be located in the upstream of 17bp from the polyadenylation site. Amino acid sequence prevalent in ribosome-inactivating proteins(RIP: Abrin A, Luffina, MAP, Ricin A., Trichosanthin and SO6) was also found in PAP gene(Ile-Gln-Met-Val-Ser-Glu-Ala-Arg-Phe-Lys-Tyr-Ile), which is determined to be the active site of RIP having enzyme activity.

Southern blot analysis was carried out to locate PAP gene in Phytolacca americana as follows: total DNA was isolated from Phytolacca americana, digested with HindIII and EcoRI and fragments produced thereof were electrophoresed on 0.8(v/w)% agarose gel. DNA was transferred to Hybond-N+ and subjected to hybridization employing 0.52kb EcoRI fragment of PAP cDNA insert as probe. In this connection, probe was prepared by nick translation kit(Promega, U.S.A.) and labelled with α-32P dATP.

Northern blot analysis was carried out as follows: poly(A) mRNA isolated from Phytolacca americana was electrophoresed on 1(v/w)% agarose gel containing 10(v/v)% formaldehyde. RNA thus fractionated was transferred to Hybond-N+ and subjected to hybridization using 0.52kb EcoRI fragment already employed in the above Southern blot analysis.

### Example 2: Manipulation of 5' and 3' site of PAP genome.

To translate PAP gene by employing CaMV 35S promoter, 5' site of PAP genome should be deleted to the site of translational initiation(ATG) as follows: PAP gene was double restricted with SacII and BamHI; and ExoIII treatment at 30°C for 10sec was followed. About 30bp deleted DNA was produced by S₁ nuclease treatment, ligated to BamHI linker by T₄ DNA ligase. DNA fragment thus obtained was transformed with competent XL1-BLUE; and sequenced to select colony produced and to locate terminator sequence in a precise manner. Deleted DNA was selected and employed for further experiment. To link 3' site of PAP gene with nopaline synthase terminator("Nos terminator"), said gene was digested with HindIII, treated with Klenow's fragment, then ligated to BamHI linker, and selection of interested gene was followed.

### Example 3: Manipulation of binary vector pBI121 and preparation of expression vector pJRM100.

To clone 1.0 kb BamHI fragment of PAP genome with binary vector pBI121(Clonetech, Lot.#6019-2), SstI restriction site near the Nos terminator was substituted with BamHI restriction site as follows: pBI121 was treated with SstI, and interested DNA was obtained after Klenow's fragment treatment and ligation to BamHI linker. 1.0kb BamHI fragment of PAP gene was fractionated on agarose gel and isolated by unidirectional electroelution. In a similar fashion, pJRM100 was prepared by ligating 11.06kb BamHI fragments of pBI121 each other by T₄ DNA ligase. pJRM100 thus obtained was transformed with CaCl₂ competent XL1-BLUE treated with CaCl₂. pBI121 containing 1.0kb BamHI fragment was selected and isolated analogously.

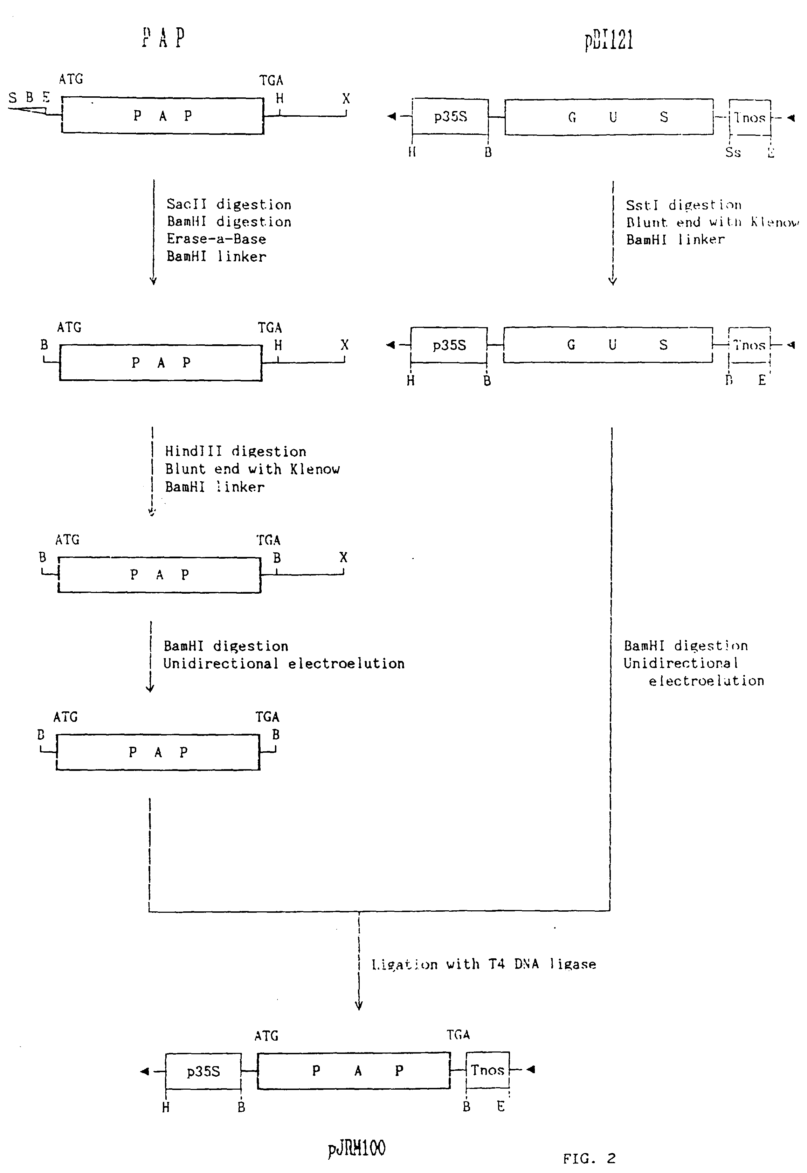

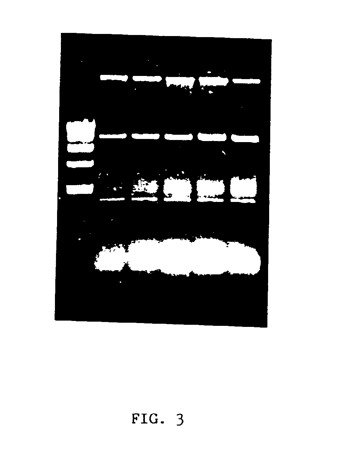

Examples 2 and 3 were illustrated in scheme of Figure 2. Figure 3 shows agarose gel electrophoresis pattern of pJRM100 digested with HindIII and XhoI(lane 1: λDNA digested with HindIII).

### Example 4: Transformation of plant cell by Agrobacterium mediator.

Freeze-thawing method was employed to transform Agrobacterium tumefaciens LBA 4404 with pJRM100 prepared in Example 3, where PAP gene expression is controlled under CaMV promotor. To select Agrobacterium tumefaciens LBA 4404 transformed with pJRM100, plasmid DNA was isolated from the Agrobacterium by quick-screening method[see: Stanton B.G. et al., Plant Molecular Biology Manual, Kluwer, Academic Publishers (1988)] and digested with BamHI. Agrobacterium tumefaciens LBA 4404 transformed with pJRM100 was deposited with Korean Collection for Type Culture (KCTC) on August 7, 1992 as deposition No. KCTC 0052BP; and claimed in the invention.

Tobacco plants, Nicotiana tabacum NC2326 and Nicotiana tabacum xanthi prepared as followings, were employed in the present invention as host organism: tobacco seed was treated with 70(v/v)% ethanol for 5min and 50(v/v)% bleaching agent for 20min respectively, then washed with distilled water 3 times and incubated on MS basic medium; and leaves grown for 1 month in incubator were chopped up in a proper size and employed as objectives for transformation. Agrobacterium tumefaciens LBA 4404 transformed with pJRM100(KCTC 0052BP), a mediator for plant cell transformation, was incubated on shaking incubator at 28°C, 200rpm for 18hrs. After cell culture, said cells were harvested, emulsified with MS medium to the concentration of 1 to 2 x 10³ cells/ml and used for tobacco plant transformation.

Transformation of tobacco leaf fragments with Agrobacterium tumefaciens LBA 4404 harboring pJRM100 were made by contacting each other for 30min; and transformant thereofs were incubated on MS solid medium comprising 1.0mg/1 BAP(benzylamino purine) and 0.1mg/1 NAA(α-Naphthalene acetic acid) at 26°C for 48hrs under dark condition. Then, incubation on MS selective medium comprising 1.0mg/1 BAP, 100mg/ℓ kanamycin and 500mg/1carbenicillin was followed to induce shoot. Incubation was made at 26°C, 300lux and daylight length was provided intermittently in 16hrs cycle; and, shoot induced therefrom was further incubated for generations, i.e., it was seeded from generation to generation every 2 weeks, under the same condition as leaf fragment incubation.

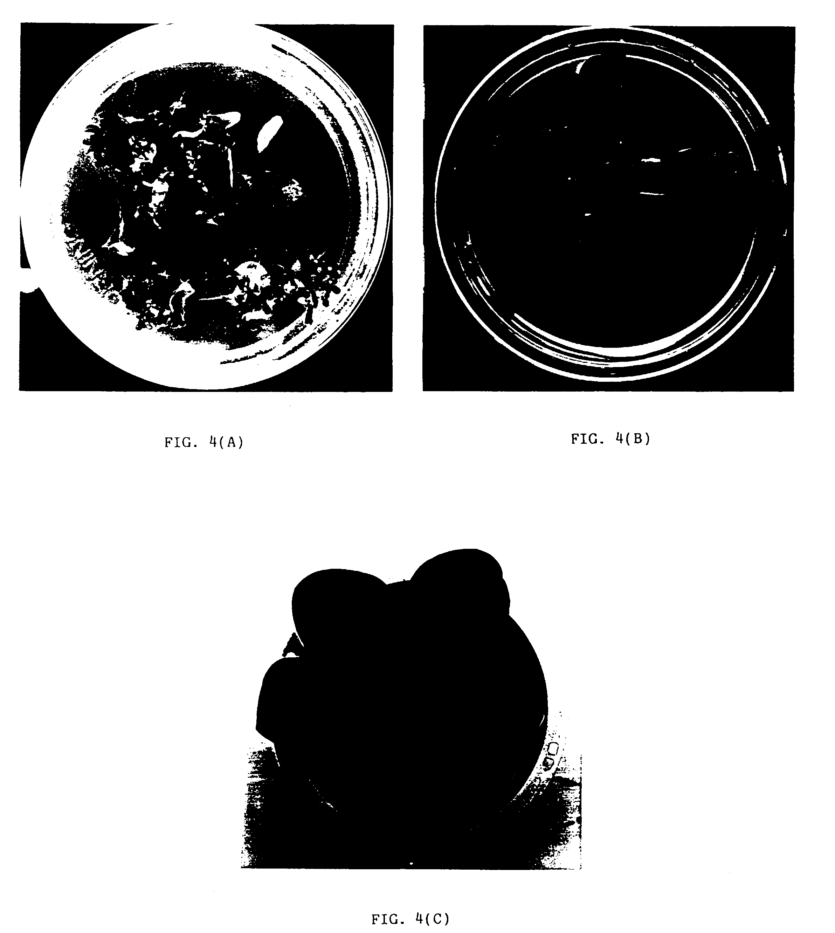

Transgenic tobacco generated on MS selective medium was subjected to root induction by incubating on MS basic medium containing 100mg/1 kanamycin and 250mg/1 carbenicillin; and tobacco thus induced was transferred to pot and adapted for later use. Figure 4 illustrates said results, wherein fig.4(A), 4(B) and 4(C) are photographs showing shoot induction after infection, root induction and pot adaptation, respectively.

### Example 5: Determination of PAP expression in transgenic tobacco.

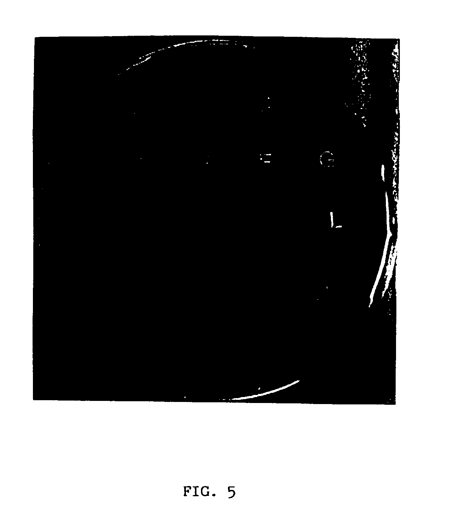

To determine PAP expression in transgenic tobacco, immunodiffusion assay employing anti-PAP antibody was carried out. After petri dish was coated with 1(v/w)% agar, mixture of 0.8(v/w)% agar and 5mg/ml anti-PAP antibody was poured on petri dish and solidified. Pores for sample loading were installed on solid agar plate. Total protein were isolated from transgenic tobacco; and samples isolated therefrom were loaded on agar plate prepared as above. Agar plate was diffused for 48 hrs, stained with dye solution, destained with destaining solution; and precipitate produced was determined. Figure 5 illustrates said results, wherein: C is total protein of Phytolacca americana L.; B is total protein of non-transformed tobacco; and, the others are total protein isolated from transformed tobacco.

### Example 6: Southern blot analysis of transgenic tobacco.

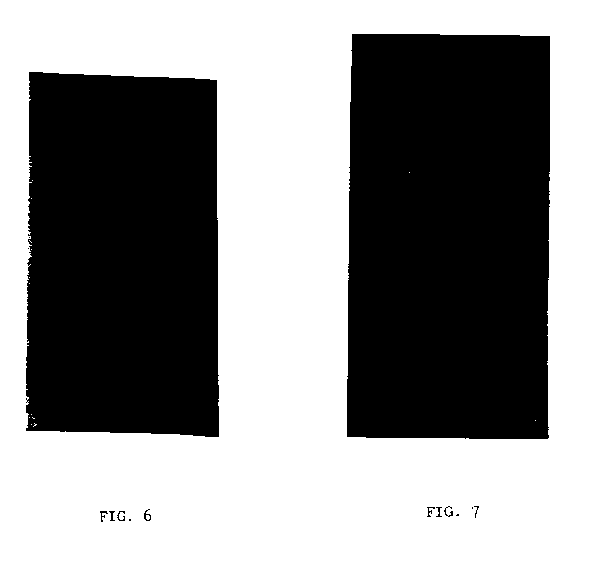

To determine proper insertion of PAP gene into tobacco gene, Southern blot analysis was carried out. Total DNA was isolated from trasgenic tobacco, digested with BamHI, and electrophoresed on 0.8%(v/w) agarose gel. DNA was transferred to Hybond-N+ (Amersham, U.K.); and hybridization was followed by employing 1.0kb BamHI fragment used in Example 3 as probe. In this connection, probe was prepared by nick translation kit(Promega, U.S.A.) and labelled with α-32P dATP. Figure 6 illustrates said results: lane 1 is BamHI digested DNA from non-transformed tobacco; lane 2, 3 and 4 are BamHI digested DNA from transformed tobacco; lane 5 is BamHI treated pJRM100, where the same band as lanes 2, 3 and 4 were determined.

### Example 7: Northern blot analysis of transgenic tobacco.

To determine transcription level in the transgenic tobacco, total RNA was isolated from transgenic tobacco, electrophoresed on 1(v/w)% agarose gel, transferred to Hybond-N+; and hybridization was carried out by employing 1.0kb BamHI fragment used in Example 3 as probe. Figure 7 illustrates said results, where no band was determined in non-transformed control(A) while one band was determined in transformed tobacco.

### Example 8: Local lesion assay of transgenic tobacco.

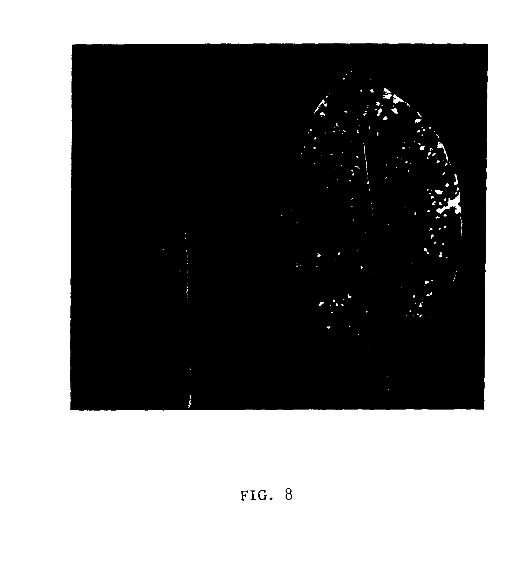

Lyophilized TMV-infected tissue(ATCC PV226) obtained from ATCC was chopped up, dissolved in phosphate buffer solution and centrifuged. Supernatant thus obtained, was formulated for virus innoculation; and administrated on test plants under 4 to 6 leaves stage. After inoculation, local lesion assay was carried out for 7 days in growth chamber. Figure 8 illustrates the said results, wherein plaque was formed in transformed tobacco(A), while no plaque was detected in non-transformed tobacco.

As clearly illustrated and demonstrated as aboves, the present invention provides a process for preparing a transgenic plant producing antiviral protein by transforming with recombinant vector for antiviral protein expression, which contains a PAP gene isolated from Phytolacca americana cDNA library and a vector for foreign protein expression. Virus-resistant plant and antiviral agent produced therefrom can also be developed in accordance with the present invention.

## Claims

1. A recombinant DNA pJRM100(KCTC (KCTC 0052BP) which expresses an antiviral protein gene in a plant cell.

2. The recombinant DNA pJRM100 of claim 1, wherein said antiviral protein is PAP (phytolacca antiviral protein).

3. The recombinant DNA pJRM 100 of claim 1, wherein said plant is tobacco.

4. Agrobacterium tumefaciens LBA 4404 transformed with recombinant DNA pJRM100 of claim 1.

5. A process for preparing an antiviral protein which comprises incubating a plant cell transformed with the recombinant DNA of claim 1.

6. The process of claim 5, wherein said antiviral protein is PAP (phytolacca antiviral protein).

7. A process for preparing a transgenic plant with viral resistance which comprises the step of transfecting with agrobacterium tumefaciens LBA 4404 according to claim 4, which is transformed with the recombinant DNA according to claim 1.

## Patentansprüche

1. Rekombinante DNA pJRM100 (KCTC 0052BP), welche ein antivirales Proteingen in einer Pflanzenzelle exprimiert.

2. Rekombinante DNA pJRM100 nach Anspruch 1, worin das antivirale Protein PAP (Phytolacca Antiviral Protein) ist.

3. Rekombinante DNA pJRM100 nach Anspruch 1, worin die Pflanze Tabak ist.

4. Agrobacterium tumefaciens LBA 4404 transformiert mit der rekombinanten DNA pJR100 nach Anspruch 1.

5. Verfahren zur Herstellung eines antiviralen Proteins, welches darin besteht, eine mit der rekombinanten DNA von Anspruch 1 transformierte Pflanzenzelle zu inkubieren.

6. Verfahren nach Anspruch 5, worin das antivirale Protein PAP (Phytolacca Antiviral Protein) ist.

7. Verfahren zur Herstellung einer transgenen Pflanze mit Virusresistenz, welches den Schritt der Transfektion mit Agrobacterium tumefaciens LBA 4404 nach Anspruch 4 umfaßt, welcher mit der rekombinanten DNA nach Anspruch 1 transformiert worden ist.

## Revendications

1. ADN pJRM100 recombinant (KCTC 0052BP) exprimant un gène de protéine antivirale dans une cellule végétale.

2. ADN pJRM100 recombinant selon la revendication 1, où ladite protéine antivirale est la protéine PAP (protéine antivirale de phytolacca).

3. ADN pJRM100 recombinant selon la revendication 1, où ledit végétal est le tabac.

4. Agrobacterium tumefaciens LBA 4404 transformé par l'ADN recombinant pJRM100 de la revendication 1.

5. Méthode de préparation d'une protéine antivirale comprenant l'incubation d'une cellule végétale transformée avec l'ADN recombinant de la revendication 1.

6. Méthode selon la revendication 5, où ladite protéine antivirale est la protéine PAP (protéine antivirale de phytolacca).

7. Méthode de préparation d'une plante transgénique avec une résistance virale qui comprend l'étape de transfection par agrobacterium tumefaciens LBA 4404 selon la revendication 4, transformé par l'ADN recombinant selon la revendication 1.
